# EUROPEAN PATENT APPLICATION

(11) **EP 3 603 657 A1**
(43) Date of publication of application: **05.02.2020**
(21) Application number: 18857493.3
(22) Date of filing: 18.09.2018
(51) Int. Cl.: A61K 36/81, A23L 33/105

(54) **COMPOSITION CONTAINING AMOMUM VILLOSUM EXTRACT FOR PREVENTION, ALLEVIATION, OR TREATMENT OF OBESITY**

(30) Priority: 21.09.2017 KR 20170121948
(71) Applicant: Corporation Ilwonbio, Iksan-si, Jeollabuk-do 54576 (KR)
(72) Inventor: KWON, Kang Beom, Jeonju-si Jeollabuk-do 54823 (KR); KIM, Ha Rim, Jeonju-si Jeollabuk-do 54866 (KR); JO, Sung Gang, Jeonju-si Jeollabuk-do 54984 (KR); LEE, Guem San, Wanju-gun Jeollabuk-do 55342 (KR)
(74) Representative: Gulde & Partner
(86) International application number: PCT/KR2018/010948
(87) International publication number: WO 2019/059606

(57) **Abstract**

The present invention relates to a composition for preventing or alleviating obesity including an *Amomum villosum* extract as an active ingredient.

The present invention relates to a composition for preventing or alleviating obesity, wherein the *Amomum villosum* extract is obtained through extraction with water and is included in a dose of 100 mg/kg to 500 mg/kg based on oral administration to a mouse.

The present invention provides a composition for preventing or alleviating obesity including an *Amomum villosum* extract having anti-obesity efficacy, wherein the *Amomum villosum* extract inhibits an increase in adipocytes in the liver, limits the size of adipocytes, inhibits the production of triglycerides, and inhibits mRNA expression *in vivo.*

## Description

### [Technical Field]

The present invention relates to a composition for preventing, alleviating, or treating obesity, which includes an *Amomum villosum* extract.

The present invention provides a composition for preventing, alleviating, or treating obesity, including an *Amomum villosum* extract, wherein the *Amomum villosum* extract is obtained through extraction with water and has an effect of preventing or alleviating obesity by inhibiting fat production in the liver, limiting the size of adipocytes, inhibiting the production of triglycerides, inhibiting adipogenic-differentiation-related mRNA expression, and inhibiting fatty-liver-inducing mRNA expression.

### [Background Art]

Obesity is a metabolic disorder caused by an imbalance between energy intake and consumption due to excessive intake of nutrition by modern people, resulting in excessive fat accumulation in the body, and is on the rise.

The obese population is gradually increasing, 1.5 billion adults were overweight or obese worldwide in 2008, and the overweight population is estimated to reach about 2.1 billion people by 2030.

In particular, obesity, which begins in childhood, shows an increase in the size and number of adipocytes and is dangerous because weight loss causes a decrease in the size of adipocytes but no decrease in the number of adipocytes during one's life.

Unlike this, obesity in adults is characterized by an increase in the size of adipocytes but no increase in the number of adipocytes. The distribution of obesity also tends to be restricted to the truncus around the abdomen.

That is, obesity in adults shows only an increase in the size of adipocytes, whereas obesity starting from childhood shows an increase in the number of adipocytes as well as an increase in the size of adipocytes, and therefore, although weight may be lost in adulthood, only the size of adipocytes temporarily decreases, and a great number of adipocytes still remains, resulting in easy recurrence of obesity.

In addition, recently, as nutritional conditions have improved and adult diseases have increased, fatty liver patients are increasing. Fatty liver is a disease caused by fat accumulation and synthesis in the liver due to excessive intake of fat and inability to perform normal fat metabolism.

70% to 80% of patients with fatty liver disease are obese, and many other patients are overweight. Thus, weight loss improves insulin sensitivity, resulting in alleviated fatty liver, and it has recently been reported that, even when only about 5% of body weight is lost, insulin resistance is improved and liver function levels are improved. Therefore, lifestyle improvement is more important than anything.

Accordingly, as the severity of obesity has recently increased, a variety of products are released as diet supplements, but side effects such as gastritis, constipation, anorexia, and the like result therefrom.

The applicant of the present invention completed the present invention, which uses an *Amomum villosum* extract, and thus does not cause side effects, does not incur toxicity even upon long-term administration, and provides an effect of alleviating obesity.

Amomum villosum refers to clumps of ripe fruits or seeds of *Amomum villosum* Loureiro var. *xanthioides* T.L. Wu et Senjen or *Amomum villosum* Loureiro, which belongs to the family Zingiberaceae, and in the present invention, *Amomum villosum* fruits were used as a raw material.

*Amomum villosum* Loureiro is mainly produced in Fujian, Guangdong, Guangxi, and Yunnan, and *Amomum villosum* Loureiro var. *xanthioides* T.L. Wu et Senjen is mainly produced in regions of Indochina, such as Cambodia, India, Laos, Myanmar, Thailand, Vietnam, and the like, and some regions of China, such as Guangxi and Yunnan (Flora of China, v24:347-356.2000). Due to the variety of production places, in ancient times, *Amomum villosum* Loureiro var. *xanthioides* T.L. Wu et Senjen was separately called *"Amomum xanthioides* Wall".

Meanwhile, the fruit of *Amomum longiligulare* T.L. Wu, which is taken as genuine in China but is taken as fake in Korea, is also mass-distributed. However, as a result of conducting research on RP-HPLC, Shen et al. (SHEN Li WANG Yang JIANG Ku et al. Comparison of HPLC Fingerprints of Amomum villosum Lour., Amomum villosum Lour. var. xanthioides T.L. Wu et Senjen and Amomum longiligulare T.L. Wu. Chinese Pharmaceutical Journal. 2016: 51(12); 1039-1043.) verified that, although three types of *Amomum villosum* exhibited high similarities in terms of ingredients, *Amomum longiligulare* T.L. Wu and *Amomum villosum* Loureiro consist of similar chemical components, whereas *Amomum villosum* Loureiro var. *xanthioides* T.L. Wu et Senjen clearly differs from the above two types, and thus a comparison of pharmacological and clinical efficacies therebetween needs to be conducted.

Therefore, to verify an effect of an *Amomum villosum* extract on preventing or alleviating obesity, a high-fat-diet-induced obesity model was used and the effect of the *Amomum villosum* extract on preventing, alleviating, or treating obesity was confirmed *in vivo* through the inhibition of fat production in the liver, limitation of the size of adipocytes, inhibition of the production of triglycerides, and the inhibition of adipogenic-differentiation-related mRNA expression and fatty-liver-inducing mRNA expression, thus completing the present invention.

Meanwhile, according to the related art, Korean Registered Patent Publication No. 10-1431610 discloses a pharmaceutical composition for preventing or treating obesity, dyslipidemia, fatty liver, or diabetes, which includes extracts from *Prunus mume Sieb. et Zucc.* and *Lithospermum erythrorhizon Sieb. et Zucc.* as active ingredients. Korean Registered Patent Publication No. 10-1206543 discloses that a composition for preventing or treating fatty liver including a *Castanea crenata* shell extract is effective.

However, the aforementioned cited references disclose the confirmation of efficacy using natural substances different from *Amomum villosum,* and thus are distinguished from the present invention.

### [Disclosure]

### [Technical Problem]

Therefore, the present invention has been made in view of the above problems, and it is an object of the present invention to provide an anti-obesity composition including an *Amomum villosum* extract, in which the anti-obesity efficacy of the *Amomum villosum* extract was confirmed using a high-fat-diet-induced obesity model.

It is an object of the present invention to provide a composition for preventing, alleviating, or treating obesity including an *Amomum villosum* extract, in which, in order to verify the effect of the *Amomum villosum* extract on preventing or alleviating obesity, a high-fat-diet-induced obesity model was used and the effect of the *Amomum villosum* extract on preventing or alleviating obesity was confirmed *in vivo* through the inhibition of fat production in the liver, limitation of the size of adipocytes, inhibition of the production of triglycerides, and the inhibition of adipogenic-differentiation-related mRNA expression and fatty-liver-inducing mRNA expression.

### [Technical Solution]

In accordance with the present invention, the above and other objects can be accomplished by the provision of a composition for preventing, alleviating, or treating obesity, which includes an *Amomum villosum* extract.

The *Amomum villosum* extract may be included in a dose of 100 mg/kg to 500 mg/kg based on oral administration to a mouse.

The *Amomum villosum* extract may be obtained through extraction with water.

The *Amomum villosum* extract may suppress the content of triglycerides in mice with obesity induced by a high-fat diet and inhibit, in the mice, the expression of peroxisome-proliferators-activated receptor γ (PPARγ), CCAAT/enhancer binding protein α (C/EBPα), fatty acid synthase (FAS), sterol regulatory element-binding protein 1 (SREBP1), tumor necrosis factor α (TNFα), and adiponectin mRNA.

The *Amomum villosum* extract may inhibit the production of triglycerides in obesity induced by a high-fat diet.

In accordance with another aspect of the present invention, there is provided a health functional food composition for alleviating obesity, which includes an *Amomum villosum* extract as an active ingredient.

### [Advantageous effects]

According to an experimental example of the present invention, the efficacy of an *Amomum villosum* extract on inhibiting fat accumulation was examined *in vivo* to confirm the anti-obesity efficacy of the *Amomum villosum* extract using a high-fat-diet-induced obesity model.

According to an experimental example of the present invention, as a result of administering an *Amomum villosum* extract to high-fat-diet-induced obesity model mice, the effect of the *Amomum villosum* extract on preventing or alleviating obesity was confirmed through the inhibition of fat accumulation in the liver, limitation of the size of adipocytes, inhibition of the production of triglycerides, and the inhibition of expression of adipogenic-differentiation-related mRNA and fatty-liver-inducing mRNA, and therefore, a composition for preventing, alleviating, or treating obesity including the *Amomum villosum* extract was developed, thus completing the present invention.

### [Description of Drawings]

FIG. 1 is a graph showing changes in the body weight of high-fat-diet-induced obesity model animals according to the dosage of an *Amomum villosum* extract.
FIG. 2 illustrates changes in liver weight and total fat content of a high-fat-diet-induced obesity model according to the dosage of an *Amomum villosum* extract.
FIG. 3 illustrates the results of observing adipose tissues in the liver of a high-fat-diet-induced obesity model according to the dosage of an *Amomum villosum* extract.
FIG. 4 illustrates the results of observing changes in adipose tissues of a high-fat-diet-induced obesity model according to the dosage of an *Amomum villosum* extract.

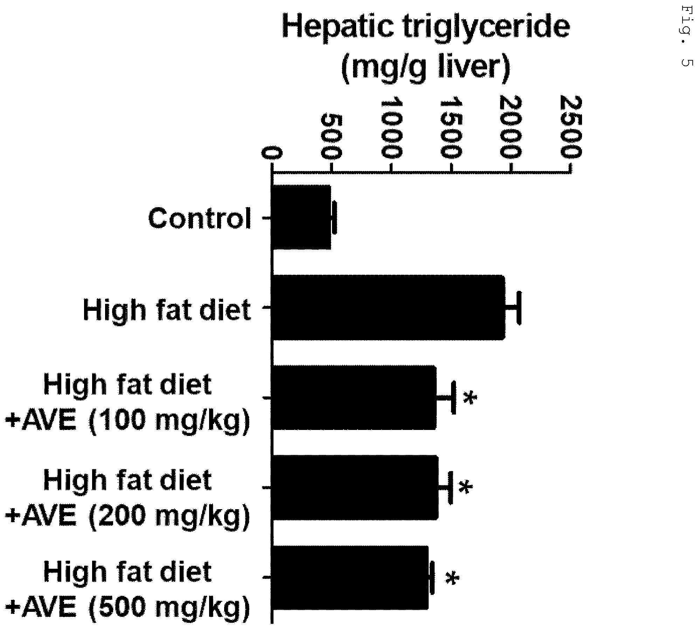

FIG. 5 is a graph showing the results of analyzing triglyceride content of a high-fat-diet-induced obesity model according to the dosage of an *Amomum villosum* extract.
FIG. 6 is a set of graphs for confirming PPARγ and C/EBPα expression levels of a high-fat-diet-induced obesity model according to the dosage of an *Amomum villosum* extract.
FIG. 7 is a set of graphs for confirming the expression levels of FAS mRNA and SREBP1 mRNA of a high-fat-diet-induced obesity model according to the dosage of an *Amomum villosum* extract.
FIG. 8 is a set of graphs for confirming the expression levels of TNFα and adiponectin of a high-fat-diet-induced obesity model according to the dosage of an *Amomum villosum* extract.

### [Best Mode]

The terms or words used in the present specification and claims should not be construed as being limited to ordinary or dictionary meanings, but should be construed as having meanings and concepts consistent with the spirit of the present invention based on the principle that an inventor can appropriately define terms to explain the invention of the inventor in the best way.

Thus, details described in examples, reference examples, and drawings of the present specification are merely the most exemplary embodiments of the present invention and do not represent all technical spirits of the present invention, and thus it should be understood that various equivalents and modifications that may replace these embodiments can be made at the time of filing of the present application.

### Experimental Example 1. Anti-obesity Effect of Amomum villosum Extract Using High-fat-diet-induced Obesity Model

### 1) Preparation of Amomum villosum Extract

*Amomum villosum* was verified for authenticity by the Department of Herbology, College of Oriental Medicine, Wonkwang University, and this process is to extract *Amomum villosum* with water to obtain an extract.

Depending on the design conditions, different solvents may be used, and the extraction temperature, extraction time, amount of solvent, residual ingredient treatment method, and the like may be designed differently according to the type of extraction solvent. As the extraction solvent, various solvents may also be used, and extractable solvents include water, ethanol, methanol, fatty oil, glycerin, horse oil, ethyl acetate, acetone, butanol, isopropanol, and the like.

Preferably, in the present invention, *Amomum villosum* was extracted with water. In the extraction process, 100 g of *Amomum villosum* was heated and reflux-extracted with 900 ml of deionized water for 3 hours, and filtered, and then a filtrate was concentrated under reduced pressure and lyophilized to obtain 9.25 g of an *Amomum villosum* water extract, followed by refrigerated storage for use in experiments. Accordingly, a composition for preventing or alleviating obesity, wherein an extract is obtained through extraction with water, may be developed.

### 2) Rearing and Diet of Experimental Animals

6-week-old male C57BL/6J mice were purchased as experimental animals (Orientbio, Seongnam, Korea) and adapted to the rearing room environment for 1 week. Mice having an average body weight of about 20 g to about 22 g were arbitrarily divided into 5 groups: a control fed normal diet, a high-fat diet control, a group administered with 100 mg/kg of the *Amomum villosum* extract plus a high-fat diet (High-fat diet+AVE(100mg/kg)), a group administered with 200 mg/kg of the *Amomum villosum* extract plus a high-fat diet (High-fat diet+AVE(200mg/kg)), and a group administered with 500 mg/kg of the *Amomum villosum* extract plus a high-fat diet (High-fat diet+AVE(500mg/kg)), and raised for 7 weeks.

The rearing room was maintained at a temperature of 18 °C to 24 °C and a relative humidity of 50% to 60%, and light/dark cycles were adjusted to be 12 hours (8:00 to 20:00). For the high-fat feed used in the present experiments, 15% of lard was additionally added to a basal diet such that about 60% of total calories was fed as fat, and the body weight of each mouse was measured once a week. Animal experiments were approved by the Wonkwang University Animal Experiment Ethics Committee, and the use and management of laboratory animals were in accordance with institutional guidelines.

### 3) Experimental Group Set-up and Sample Administration

The experimental animals were weighed after the acclimation period. Experimental groups were divided into a control group, a high-fat diet group, a group administered with high-fat feed + 100 mg/kg (*Amomum villosum* extract, AVE 100 mg/kg group), a group administered with high-fat feed + 200 mg/kg of the *Amomum villosum* extract (*Amomum villosum* extract, AVE 200 mg/kg group), and a group administered with high-fat feed + 500 mg/kg of the *Amomum villosum* extract (*Amomum villosum* extract, AVE 500 mg/kg group), and 10 mice per group were used in the experiments, and the *Amomum villosum* extract was administered using an oral zonde needle daily for 7 weeks according to predetermined concentrations.

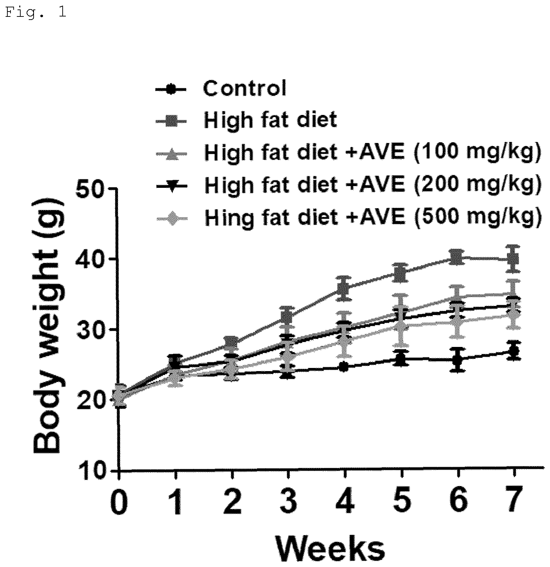

FIG. 1 is a graph showing changes in the body weight of high-fat-diet-induced obesity model animals according to the dosage of an *Amomum villosum* extract.

As a result, it was confirmed that the larger the dosage of the *Amomum villosum* extract, the smaller the body weight. That is, a composition for preventing, alleviating, or treating obesity, wherein the *Amomum villosum* extract is included in a dose of 100 mg/kg to 500 mg/kg based on oral administration to a mouse, may be prepared.

### 4) Biomarker Analysis and Autopsy

Body weight was measured weekly after the start of the experiment, and for autopsy, blood was collected from the abdominal vein after anesthesia with ether to be used for blood analysis, and the liver and adipose (epididymal fat) tissues were extracted and weighed.

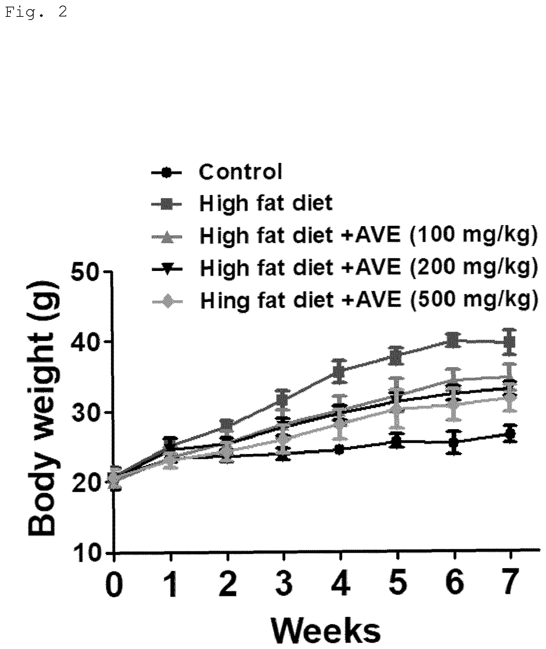

FIG. 2 illustrates changes in liver weight and total fat content of a high-fat-diet-induced obesity model according to the dosage of an *Amomum villosum* extract.

As a result, it was confirmed that the greater the dosage of the *Amomum villosum* extract, the smaller the weights of the liver and adipose tissues.

### 5) Histological Analysis

The extracted liver and adipose tissues were fixed in a 10% formalin solution, and the samples were trimmed and post-fixed again in a 10% formalin solution. Subsequently, the samples were embedded in paraffin, sectioned to a thickness of 5 µm, finely cut, and then stained with hematoxylin-eosin. The stained liver and adipose tissues were observed and photographed using an Olympus BX50 F4 optical microscope (Olympus, Japan).

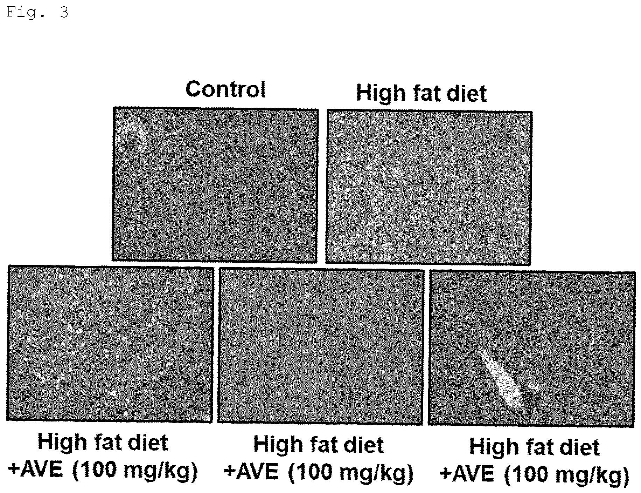

FIG. 3 illustrates the results of observing adipose tissues in the liver of a high-fat-diet-induced obesity model according to the dosage of an *Amomum villosum* extract.

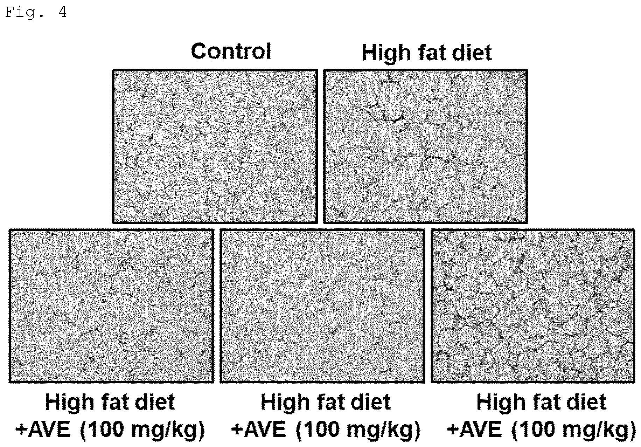

FIG. 4 illustrates the results of observing changes in adipose tissues of a high-fat-diet-induced obesity model according to the dosage of an *Amomum villosum* extract.

As a result, it was confirmed that, as the dosage of the *Amomum villosum* extract increased, the amount of fat in the liver was decreased and the size of adipose tissue was decreased.

### 6) Total RNA Isolation

After the experiment was completed, total RNA was isolated from the extracted liver and adipose tissues using a TRIzol reagent (Life Technologies, UK) according to the method provided by the manufacturer.

Each tissue was chopped and then dissolved in 1,000 µl of RNAzol B, and then 200 µl of chloroform was added thereto to allow a reaction to occur therebetween in ice for 5 minutes. After the reaction was completed, the reaction product was centrifuged at 4 °C and 13,000 rpm for 20 minutes, and 500 µl of a supernatant was transferred to a new tube.

500 µl of isopropanol was added to the supernatant and mixed, and then a reaction was allowed to occur therebetween on ice for 30 minutes. After completion of the reaction, the reaction product was centrifuged at 4 °C and 13,000 rpm for 20 minutes, and a precipitate was washed twice with 75% EtOH. The washed RNA was dried and then lysed with 20 µl of DEPC water, and for quantification, absorbance was measured using a spectrophotometer.

### 7) Real-Time Reverse Transcriptase Polymerase Chain Reaction (Real Time RT-PCR)

A reverse transcription reaction was performed using 2 µg of total RNA and a PrimeScript™ reagent kit (Perfect Real Time, TaKaRa Bio Inc.) according to the method provided by the manufacturer.

For the reverse transcription reaction, a reaction solution containing total RNA (2 µg), oligo d(T)primer (25 pmol), PrimeScript RT enzyme Mix I, and 5X PrimeScript buffer was allowed to react at 37 °C for 15 minutes and at 85 °C for 5 hours to thereby synthesize cDNA.

The real-time reverse transcriptase polymerase chain reaction was performed on 10-fold diluted cDNA using a Power SYBY Green PCR Master Mix. The mRNA level of each expressed gene was calculated as an amount relative to GAPDH using LightCycler System software (Roche). The sequences of used primers are shown in Table 1 below.

**[Table 1]**

| **Sequences and Accession Numbers for Primer, Forward and Reverse, Used in Real-Time PCR.** | | |
|---|---|---|
| Gene | Sequence for Primers | Accession no. |
| GAPDH | Forward: CGTCCCGTAGACAAAATGGTReverse: TTGATGGCAACAATCTCCAC | NM_008084 |
| FAS | Forward: GCG ATG AAG AGC ATG GTT TAG | NM_007988 |
| | Reverse: GGC TCA AGG GTT CCA TGT T | |
| SREBP1 | Forward: GGA GCC ATG GAT TGC ACA | NM_011480 |
| | TTReverse: GGC CCG GGA AGT CAC TGT | |
| Adiponectin | Forward: ACG TCA TCT TCG GCA TGA | NM_009605 |
| | CTReverse: CTC TAA AGA TTG TCA GTG GAT CTG | |
| TNFα | Forward: AGG GTC TGG GCC ATA GAA CT | NM_013693 |
| | Reverse: CCA CCA CGC TCT TCT GTC TAC | |
| PPARγ | Forward: GAAAGACAACGGACAAATCACCReverse: GGGGGTGATATGTTTGAACTTG | NM_011146 |
| C/EBPα | Forward: TTGTTTGGCTTTATCTCGGCReverse: CCAAGAAGTCGGTGGACAAG | NM_007678 |

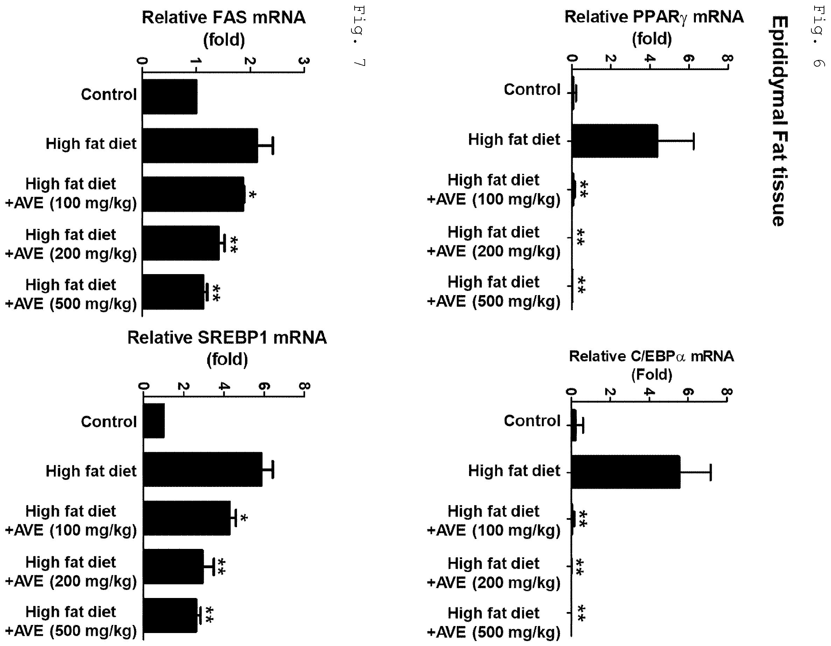

FIG. 6 is a set of graphs for confirming PPARγ and C/EBPα expression levels of a high-fat-diet-induced obesity model according to the dosage of an *Amomum villosum* extract.

FIG. 7 is a set of graphs for confirming the expression levels of FAS mRNA and SREBP1 mRNA of a high-fat-diet-induced obesity model according to the dosage of an *Amomum villosum* extract.

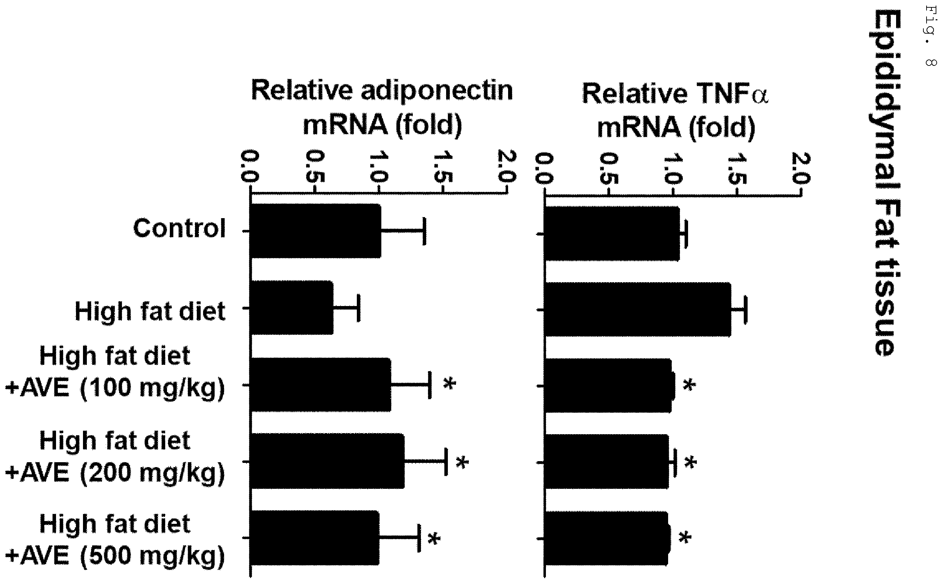

FIG. 8 is a set of graphs for confirming the expression levels of TNFα and adiponectin of a high-fat-diet-induced obesity model according to the dosage of an *Amomum villosum* extract.

As a result, it was confirmed that the expression of peroxisome-proliferators-activated receptor γ (PPARγ), CCAAT/enhancer binding protein α (C/EBPα), fatty acid synthase (FAS), sterol regulatory element-binding protein 1 (SREBP1), tumor necrosis factor α (TNFα), and adiponectin mRNA was inhibited in mice with obesity induced by a high-fat diet according to the dosage of the *Amomum villosum* extract, which is anticipated to inhibit the differentiation and expression of adipocytes and fatty liver expression.

A composition for preventing, alleviating, or treating obesity, which suppresses the content of triglycerides in mice with obesity induced by a high-fat diet and inhibits, in the mice, the expression of peroxisome-proliferators-activated receptor γ (PPARγ), CCAAT/enhancer binding protein α (C/EBPα), fatty acid synthase (FAS), sterol regulatory element-binding protein 1 (SREBP1), tumor necrosis factor α (TNFα), and adiponectin mRNA, may be prepared.

### 8) Liver Triglyceride Analysis

The content of hepatic triglycerides was measured as follows. The liver tissues were homogenized in a chloroform-methanol solution (2:1, v/v), allowed to react at room temperature for 1 hour, and then centrifuged, and then an organic solvent layer was dried for 24 hours. The dried organic solvent layer was dissolved in ethanol, and then the content of hepatic triglycerides was examined using a TG kit (AM 157S-K and AM 202-K, Asan Pharmaceutical, Korea) and normalized to a protein concentration.

The inhibition of triglyceride production according to the dosage of the *Amomum villosum* extract can be confirmed in mice with obesity induced by a high-fat diet.

Since the *Amomum villosum* extract inhibited triglyceride production in mice with obesity induced by a high-fat diet, a composition for preventing, alleviating, or treating obesity was developed therethrough.

### 9) Statistical Analysis

All experimental results were calculated as mean ± standard error. For statistical analysis according to statistical significance verification in each group, an one-way analysis of variance test (ANOVA) and Duncan's post-hoc comparison were performed, and p < 0.05 was taken as significant (SPSS V12., SPSS Inc, Chicago, IL, USA).

## Claims

1. A health functional food composition for preventing or alleviating obesity induced by a high-fat diet, the health functional food composition comprising an *Amomum villosum* extract as an active ingredient,
wherein the *Amomum villosum* extract is obtained through extraction with water and is included in a dose of 100 mg/kg to 500 mg/kg based on oral administration to a mouse, and
the *Amomum villosum* extract suppresses a content of triglycerides in mice with obesity induced by a high-fat diet and inhibits, in the mice, the expression of peroxisome proliferators-activated receptor γ (PPARγ), CCAAT/enhancer binding protein α (C/EBPα), fatty acid synthase (FAS), sterol regulatory element-binding protein 1 (SREBP1), tumor necrosis factor α (TNFα), and adiponectin mRNA.
